(19) Europäisches Patentamt
European Patent Office
Office européen des brevets

(11) Veröffentlichungsnummer: **0 006 128**
**A1**

(12) # EUROPÄISCHE PATENTANMELDUNG

(21) Anmeldenummer: **79101438.4**

(22) Anmeldetag: **11.05.79**

(51) Int. Cl.³: **C 07 D 209/16**, A 61 K 31/40

(30) Priorität: **08.06.78 DE 2825192**

(43) Veröffentlichungstag der Anmeldung: **09.01.80 Patentblatt 80/1**

(84) Benannte Vertragsstaaten: **AT BE CH DE FR GB IT LU NL SE**

(71) Anmelder: **Dr. Karl Thomae GmbH, Postfach 720, D-7950 Biberach (Riss) (DE)**

(72) Erfinder: **Landgraf, Claus Adolf, Dr., Schubertweg 3, D-7950 Biberach 1 (DE)**
Erfinder: **Püschmann, Sigfrid, Dr. Dipl-Biologe, Göserweg 8, D-7950 Biberach 1 (DE)**
Erfinder: **Engel, Wolfhard, Dr. Dipl.-Chem., Mozartstrasse 13, D-7950 Biberach 1 (DE)**
Erfinder: **Seeger, Ernst, Dr. Dipl.-Chem., Alpenstrasse 39, D 7950 Biberach 1 (DE)**

(54) **2,3-Dihydro-2-methyl-3-(2-(dimethylamino)-propyl)-3-phenyl-1H-indol-1-carboxaldehyd, Verfahren zu dessen Herstellung und diese Verbindung enthaltende Arzneimittel.**

(57) Die Erfindung betrifft ein Diastereomer des 2,3-Dihydro-2-methyl-3-[2-(dimethylamino)-propyl]-3-phenyl-1H-indol-1-carboxaldehyds der Formel

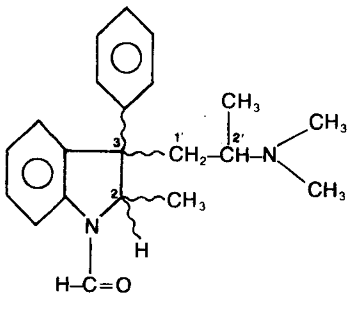

(I)

und dessen beide Enantiomere, Salze dieser Verbindungen, Verfahren zu ihrer Herstellung ausgehend vom 2,3-Dihydro-2-methyl-3-[2-(dimethylamino)-propyl]-3-phenyl-1H-indol, welches formyliert, oder von 2-Methyl-3-[2-(dimethylamino)-propyl]-3-phenyl-3H-indol, welches reduktiv formyliert wird, des weiteren diese Verbindungen enthaltende Arzneimittel. Die Verbindungen wirken antitussiv, das Racemat und das (–)-Entantiomere gleichzeitig auch broncholytisch.

EP 0 006 128 A1

0006128

Case 5/737
Dr.Pu/ro

DR. KARL THOMAE GMBH, BIBERACH AN DER RISS

# 2,3-Dihydro-2-methyl-3-[2-(dimethylamino)-propyl]-3-phenyl-1H-indol-1-carboxaldehyd, Verfahren zu dessen Herstellung und diese Verbindung enthaltende Arzneimittel

Die Erfindung betrifft eines der möglichen Diastereomeren des 2,3-Dihydro-2-methyl-3-[2-(dimethylamino)-propyl]-3-phenyl-1H-indol-1-carboxaldehyds der Formel I,

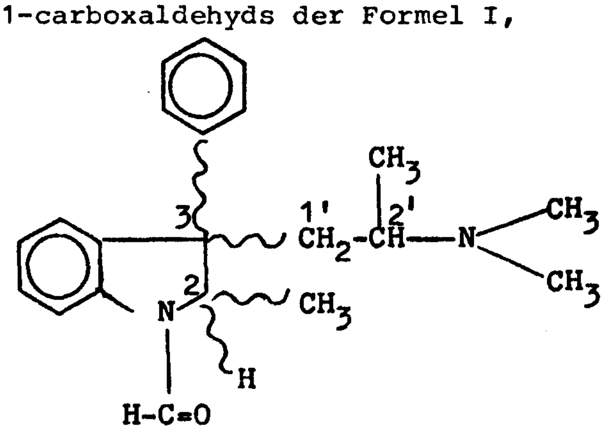

(I)

dessen beide Enantiomere sowie deren physiologisch verträgliche Salze mit anorganischen oder organischen Säuren, Verfahren zur Herstellung dieser Verbindungen und diese Verbindungen enthaltende Arzneimittel.

Bei dem beanspruchten Diasteromeren dürfte der 2-Methyl- zum 3-Phenyl-Rest in trans-Stellung stehen. Das beanspruchte Diastereomere vom Schmelzpunkt 71-72°C wird charakterisiert durch folgende $^{1}H$-NMR-Daten:

<u>$^{1}H$-NMR</u> ($d_6$-DMSO, 90 MHz, TMS als innerer Standard):

a) <u>400 K:</u> $\delta$ = 8,63 (1H-s, CHO); 7,0 - 7,7 (9H-m, aromat. H); 4,65 (1H-q, 2-H, J = 6 Hz); 1,76 - 2,5 (3H-m, Protonen in 1' und 2'); 2,03 (6H-s, $N(CH_3)_2$); 1,38 (3H-d, 2-$CH_3$, J = 6Hz); 0,66 (3H-d, $CH_3$ der Seitenkette, J = 6 Hz)

b) <u>300 K:</u> $\delta$ = 8,48 und 8,82 (jeweils s, zusammen 1H); 7,6 - 8,05 (1H-m, aromat. H); 7,0 - 7,5 (8H-m, aromat. H); 4,52 und 4,86 (jeweils q, zusammen 1H, J = 6Hz); 1,7 - 2,75 (9H-m); 1,37 und 1,22 (jeweils d, zusammen 3H, J = 6 Hz); 0,5 - 0,8 (3H-m, evt. Doppeldublett)

Die Verbindungen besitzen wertvolle therapeutische Eigenschaften, sie wirken stark antitussiv.

Das Diasteromere bzw. dessen Enantiomere lassen sich nach an sich bekannten Methoden darstellen durch Formylieren von dem entsprechenden Diasteromeren des 2,3-Dihydro-2-methyl-3-/2-(dimethylamino)-propyl/-3-phenyl-1H-indols der Formel II,

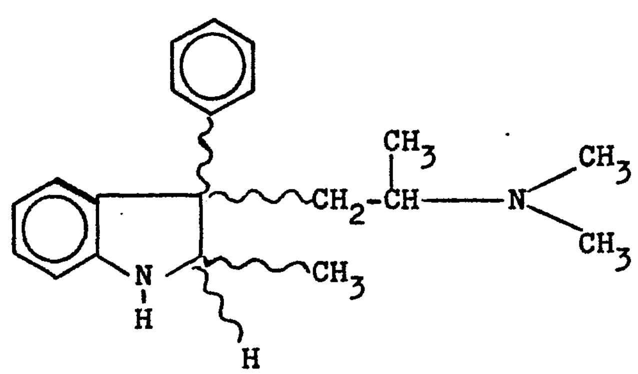

(II)

bzw. von dessen Enantiomeren und zwar

a) durch direktes Umsetzen mit Ameisensäure;

b) durch Einwirkung des gemischten Anhydrids aus Essigsäure und Ameisensäure;

c) durch Umsetzung mit Ameisensäureestern;

d) durch Umsetzung mit Ameisensäure unter Verwendung von Carbodiimiden;

e) durch Umsetzung mit dem aus Ameisensäure und Carbonyldiimidazol erhältlichen Ameisensäureimidazolid;

f) durch Umsetzung der Verbindung der Formel III

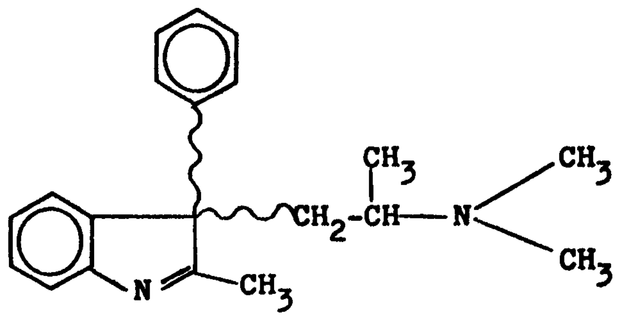

(III)

vom Schmelzpunkt 102°C mit Ameisensäure unter hydrierenden Bedingungen.

Die Umsetzung der Verbindung der Formel II mit Ameisensäure erfolgt mit oder ohne zusätzliche inerte Lösungsmittel, wie z.B. Benzol, Toluol oder Chloroform, bei Temperaturen zwischen 50 und 150°C, bevorzugt bei 100°C.

Die Formylierung mittels des gemischten Anhydrids aus Essigsäure und Ameisensäure kann mit und ohne Lösungsmittel durchgeführt werden. Als eventuelle Lösungsmittel kommen inerte, wasserfreie Lösungsmittel wie Chloroform, Benzol oder Dioxan in Frage. Die Umsetzung erfolgt bei Raumtemperatur oder bei Temperaturen bis zu 50°C, bevorzugt bei 25 bis 30°C.

Zur Formylierung mit Ameisensäureestern kommen solche Ester in Betracht, deren alkoholische Komponente bis sieben Kohlenstoffatome enthält; man arbeitet bei Temperaturen zwischen 50 und 150°C, bevorzugt um 120°C und unter Verwendung eines Autoklaven. Überschüssiger Ester dient zugleich als bevorzugtes Lösungsmittel.

Zwecks Formylierung der Verbindung der Formel II mit Ameisensäure in Gegenwart von Carbodiimiden setzt man die Reaktionsteilnehmer bei -30 bis +50°C, bevorzugt bei Raumtemperatur in einem inerten Lösungsmittel, wie z.B. Aceton, Tetrahydrofuran oder Methylenchlorid, um.

Das zur Formylierung mit Ameisensäureimidazolid erforderliche Reagens bereitet man in situ aus Ameisensäure und Carbonyl-N,N'-diimidazol unter Verwendung von trockenen, inerten Lösungsmitteln, wie z.B. Methylenchlorid, Dioxan, Dimethylformamid. Die Umsetzung wird bei -30 bis +50°C, bevorzugt bei Raumtemperatur, durchgeführt.

Die reduktive Formylierung der Verbindung der Formel III läßt sich in Ameisensäurelösung unter Verwendung von Edelmetallkatalysatoren, wie z.B. Palladium-Kohle, bei einem Wasserstoffdruck von 1 bis 50 bar und bei einer Temperatur von 20 bis 50°C, bevorzugt bei 30°C, durchführen. Es hat sich als zweckmäßig erwiesen, das Reaktionsgemisch anschließend an die Hydrierung zum Sieden zu erhitzen.

Das Racemat der Formel I und dessen Enantiomere lassen sich nach an sich bekannten Methoden in ihre physiologisch verträglichen Salze mit anorganischen oder organischen Säuren überführen; als Säuren eignen sich beispielsweise Salzsäure, Schwefelsäure, Weinsäure, Milchsäure.

Die Ausgangsverbindung der Formel II wurde veröffentlicht in der Deutschen Offenlegungsschrift 1 931 477 die Verbindung der Formel III in der Deutschen Offenlegungsschrift 1 470 370.

Die Ausgangsverbindung der Formel II läßt sich aus der Verbindung der Formel III durch katalytische Hydrierung in 1n-salzsaurer Lösung und unter Verwendung von Palladium auf Kohle als Katalysator erhalten. Die dabei erhaltene Verbindung der Formel II, die dann anschließend formyliert wird, schmilzt bei 122 bis 124°C (Äther) und hat folgendes NMR-Spektrum:
$^{1}$H-NMR ($CDCL_3$ nach Austausch mit $D_2O$, 80 MHz, TMS als innerer Standard, Raumtemperatur):

$\delta$ = 6,7 - 7,7 (9 H-m, aromat. H); 3,67 (1H-q, 2-H, J = 6,5 Hz); 2,08 (6 H-s, $N(CH_3)_2$); 1,8 - 2,9 (3 H-m, Protonen in 1' und 2'); 1,26 (3 H-d, 2-$CH_3$, J = 6,5 Hz); 0,66 (3 H-d, $CH_3$ der Seitenkette, J = 6,5 Hz).

Die Verbindung der Formel III wird durch Umsetzung des Phenylhydrazons von 2-Dimethylamino-4-phenyl-hexanon-5 mit wasserfreiem Zinkchlorid in Äthanol bei Temperaturen bis zum Siedepunkt des Reaktionsgemisches hergestellt. Die Verbindung der Formel III zeigt folgendes NMR-Spektrum:
$^{1}$H-NMR ($CDCl_3$, 80 MHz, TMS als innerer Standard):

$\delta$ = 7,65 (1 H-d, 7-H, J = 7 Hz); 7,0 - 7,5 (8 H-m, aromat. H); 2,12 (3 H-s, 2-$CH_3$); 2,06 (6 H-s; $N(CH_3)_2$); 1,7 - 2,9 (3 H-m; Protonen in 1' und 2'); 0,64 (3 H-d, $CH_3$ der Seitenkette, J = 6,5 Hz).

Das Diastereomere der Formel I und dessen Enantiomere und deren Salze mit physiologisch verträglichen Säuren besitzen wertvolle pharmakologische Eigenschaften. Sie wirken im Tierversuch insbesondere antitussiv, das Racemat und das (-)-Enantiomere zugleich broncholytisch.

Die Hustenversuche wurden an wachen weißen Ratten (Geschlecht: männlich) durchgeführt. Die Hustenprovokation erfolgte durch Inhalation eines 7,5 %igen Zitronensäuresprays über 2 Minuten. 10 Tiere pro Dosis erhielten die Substanz als Tyloseaufschwemmung peroral appliziert. Die Bewertung erfolgte aus der durchschnittlichen prozentualen Abnahme der Zahl der Hustenstöße 30 Minuten nach Applikation gegenüber den Leerwerten. (Weitere methodische Angaben: ENGELHORN und PÜSCHMANN, Arzneim.-Forsch. 13, 474-480,(1963)). Die $ED_{50}$ ist die Dosis, nach deren Verabreichung die Zahl der Hustenstöße durchschnittlich um die Hälfte vermindert wird, die Berechnung erfolgte nach der Methode von Litchfield und Wilcoxon. Es wurde für das Racemat eine $ED_{50}$ von 3,9 mg/kg per os gefunden.

Nach der Methode von Domenjoz (Arch. Exp. Path. Pharmacol. 215, 191 (1952)) wurden das Racemat und das (+)-Enantiomere auf die antitussive Wirkung an der Katze geprüft und zeigten im Dosisbereich 0,5 bis 2 mg/kg i.v. eine deutliche hustenstillende Wirkung.

Die broncholytische Wirkung wurde an Meerschweinchen nach der Methode von KONZETT und RÖSSLER (Arch. exp. Path. Pharmakol. 195, 71, (1940)) geprüft. Die Herabsetzung der durch Acetylcholin hervorgerufenen Bronchialspasmen um 50 % wurde durch i.v.-Injektion von 1,5 mg/kg des Racemats erreicht, beim (-)-Enantiomeren reichten bereits 1 mg/kg aus, um etwa den gleichen Effekt zu erreichen.

Für die Bestimmung der akuten Toxizität wurden Mäuse beider Geschlechter mit einem Körpergewicht von 20 g verwendet. 10 Tiere pro Dosis erhielten die Substanz einmalig appliziert. Die Berechnung der $DL_{50}$, der Dosis, nach deren Verabreichung 50 % der Tiere innerhalb der Beobachtungszeit verstarben, erfolgte nach der Methode von LITCHFIELD und WILCOXON nach einer Beobachtungszeit von 14 Tagen:

Racemat $DL_{50}$ p.o.: 545 mg/kg
$DL_{50}$ i.v.: 66 mg/kg,

(+)-Enantiomeres $DL_{50}$ p.o.: ~ 500 mg/kg;
(-)-Enantiomeres p.o.: ~ 560 mg/kg.

Die folgenden Beispiele sollen die Erfindung näher erläutern:

Beispiel 1

Rac. 2,3-Dihydro-2-methyl-3-/2-(dimethylamino)-propyl/-3-phenyl-1H-indol-1-carboxaldehyd

46,0 g trans-2,3-Dihydro-2-methyl-3-/2-(dimethylamino)-propyl/-3-phenyl-1H-indol (Schmp.: 122-124°C) löst man in 500 ml Toluol und gibt unter kräftigem Rühren 45 ml 99 %ige Ameisensäure hinzu. Unter weiterem kräftigem Rühren erhitzt man 3 Stunden unter Rückfluß. Dann fügt man zusätzlich noch 20 ml Ameisensäure hinzu und kocht erneut 2 Stunden unter Rückfluß. Man destilliert im Wasserstrahlvakuum die flüchtigen Bestandteile ab, nimmt den öligen Rückstand in wenig Wasser auf und überführt das Formiat mit kalter, 20%iger Kaliumcarbonat-Lösung in die freie Base.

Unter Kühlung gibt man zusätzlich festes Kaliumcarbonat hinzu und schüttelt den Ansatz dreimal mit je 200 ml Methylenchlorid aus. Nach dem Trocknen der organischen Phase mit Natriumsulfat filtriert man die Lösung, engt sie ein und nimmt den öligen Rückstand in 80 ml 60°C warmem Hexan auf. Nach dem Abkühlen der Lösung auf Raumtemperatur impft man mit einigen Kristallen der in einem Reagensglas zur Kristallisation gebrachten Base an. Man läßt 2 Stunden unter Eiskühlung stehen, saugt die Kristalle scharf ab und wäscht mit wenig eiskaltem Hexan nach.

Ausbeute: 94 % der Theorie, Schmelzpunkt: 71-72°C.

46,0 g der Base löst man in 100 ml Essigsäureäthylester-Aceton (9:1) und fügt eine Lösung von 13,5 g Methansulfonsäure in 50 ml des gleichen Gemisches zu. Man nutscht den ausfallenden Kristallbrei eiskalt ab, kocht das Kristallisat dann mit Essigsäureäthylester unter kräftigem Rühren aus, saugt die Kristalle

noch heiß ab und trocknet bei 100°C im Vakuum.
Schmp.: 169-171°C.

Entsprechend wurde das Hydrochlorid erhalten,
Schmp.: 178-180°C.

## Beispiel 2

Rac. 2,3-Dihydro-2-methyl-3-/2-(dimethylamino)-propyl/-3-phenyl-1H-indol-1-carboxaldehyd

30,0 g Essigsäureanhydrid und 18,0 g Ameisensäure (99 %ig) rührt man 3 Stunden bei 50°C. Nach dem Erkalten gibt man das gemischte Anhydrid zu 4,0 g trans-2,3-Dihydro-2-methyl-3-/2-(dimethylamino)-propyl/-3-phenyl-1H-indol (Schmp.: 122-124°C), rührt 4 Stunden bei Zimmertemperatur, zersetzt die Reaktionslösung mit Eis, macht die Lösung mit Kaliumcarbonat alkalisch und schüttelt die erhaltene Base mit Äther aus. Die abgetrennte Ätherphase trocknet man mit Kaliumcarbonat, filtriert ab und verdampft die flüchtigen Anteile im Wasserbad. Den öligen Rückstand löst man in Hexan und einigen Tropfen Äther und läßt unter Kühlung die Base auskristallisieren.
Ausbeute: 4,0 g, Schmelzpunkt: 71-72°C.

## Beispiel 3

Rac. 2,3-Dihydro-2-methyl-3-/2-(dimethylamino)-propyl/-3-phenyl-1H-indol-1-carboxaldehyd

8,0 g Dicyclohexylcarbodiimid löst man in 100 ml trockenem Aceton, fügt 2 ml Ameisensäure (99 %ig) hinzu und versetzt den Reaktionsansatz mit einer Lösung von 3,0 g trans-2,3-Dihydro-

2-methyl-3-/2-(dimethylamino)-propyl/-3-phenyl-1H-indol (Schmp. 122-124°C) in 20 ml trockenem Aceton. Die gut durchgerührte Lösung läßt man 4 Stunden bei Raumtemperatur stehen. Man filtriert vom entstandenen Dicyclohexylharnstoff ab und dampft das Aceton-Filtrat ein. Den Rückstand verteilt man zwischen kalter, 20 %iger Kaliumcarbonatlösung und Äther, die abgetrennte Ätherschicht wird nach dem Trocknen mit wasserfreiem Natriumsulfat filtriert und eingeengt. Den öligen Rückstand löst man in 5 ml Hexan und versetzt mit einigen Tropfen Äther. Der nach Eiskühlung erhaltene Niederschlag wird abgesaugt und mit kaltem Hexan gewaschen.
Ausbeute: 2,1 g, Schmelzpunkt: 71-72°C.

Beispiel 4

Rac. 2,3-Dihydro-2-methyl-3-/2-(dimethylamino)-propyl/-3-phenyl-1H-indol-1-carboxaldehyd

5,0 g 2,3-Dihydro-2-methyl-3-/2-(dimethylamino)-propyl/-3-phenyl-1H-indol (Schmp.: 122-124°C) werden in 100 ml Ameisensäureäthylester gelöst und im Druckgefäß 12 Stunden auf 120°C erhitzt. Nach dem Abkühlen überführt man das Reaktionsgemisch in einen Destillationskolben und destilliert die flüchtigen Bestandteile im Wasserstrahlvakuum ab. Das zurückbleibende Öl verteilt man zwischen kalter, gesättigter Kaliumcarbonat-Lösung und Äther. Die Ätherschicht trennt man ab, trocknet sie mit Natriumsulfat, filtriert sie und dampft den Äther ab. Den Rückstand nimmt man in 10 ml Hexan auf, das mit 5 Tropfen Äther versetzt ist. Nach Eiskühlung fällt die Base aus.
Ausbeute: 4,5 g, Schmelzpunkt: 71-72°C.
Das daraus hergestellt Methansulfonat hat einen Schmelzpunkt von 169-171°C.

Die gleiche Verbindung vom Schmelzpunkt 71-72°C erhält man in der gleichen Ausbeute bei Verwendung von Ameisensäuremethylester, Ameisensäure-n-propylester, Ameisensäure-n-butylester, Ameisensäure-n-amylester und Ameisensäurebenzylester an Stelle von Ameisensäureäthylester.

Beispiel 5

Rac. 2,3-Dihydro-2-methyl-3-[2-(dimethylamino)-propyl]-3-phenyl-1H-indol-1-carboxaldehyd

5,0 g 2-Methyl-3-[2-(dimethylamino)-propyl]-3-phenyl-3H-indol (Verbindung der Formel III mit dem Schmelzpunkt 102°C) löst man in 80 ml Ameisensäure (99 %ig) und hydriert in Gegenwart von 1,0 g 10%igem Palladium-Kohle-Katalysator bei 35°C und 5 bar Wasserstoffdruck. Nach vollständiger Wasserstoffaufnahme filtriert man vom Katalysator ab und erwärmt das Filtrat 3 Stunden zum Sieden. Anschließend dampft man die Lösung ein, löst den Rückstand in Wasser, versetzt die Lösung mit Kaliumcarbonat und äthert die Base aus. Diesselbe wird, wie in Beispiel 4 beschrieben, isoliert.
Ausbeute: 2,5 g, Schmelzpunkt: 71-72°C.

Beispiel 6

Rac. 2,3-Dihydro-2-methyl-3-[2-(dimethylamino)-propyl]-3-phenyl-1H-indol-1-carboxaldehyd

Man löst 2,0 g Carbonyldiimidazol in 40 ml wasserfreiem Methylenchlorid und versetzt mit der Lösung von 1,0 g 99 %iger Ameisensäure in 5 ml Methylenchlorid. Man läßt 1 Stunde bei

Raumtemperatur stehen, tropft dann 2,8 g 2,3-Dihydro-2-methyl-3-/2-(dimethylamino)-propyl/-3-phenyl-1H-indol, gelöst in 10 ml Methylenchlorid, zu und läßt 24 Stunden bei Raumtemperatur stehen. Dann dampft man die Lösung ein, verteilt den Rückstand zwischen kalter Kaliumcarbonatlösung und Äther und isoliert die gewünschte Base, wie in Beispiel 4 beschrieben.
Ausbeute: 1,5 g, Schmelzpunkt: 71-72°C.

Beispiel 7

(+)-2,3-Dihydro-2-methyl-3-/2-(dimethylamino)-propyl/-3-phenyl-1H-indol-1-carboxaldehyd

Man verfährt wie im Falle des Beispiels 1 mit dem Unterschied, daß man (+)-2,3-Dihydro-2-methyl-3-/2-(dimethylamino)-propyl/-3-phenyl-1H-indol (ölige Base, beschrieben in der Deutschen Offenlegungsschrift 1 931 477,Beispiel 2b) anstelle des Racemats einsetzt.

Die anfallende optisch aktive Base wird in das Methansulfonat übergeführt, welches einen Schmelzpunkt von 178-180°C hat.

$[\alpha]_D^{20}$ = + 96°C (c = 1; 1 dm; Äthanol rein)

Beispiel 8

(-)-2,3-Dihydro-2-methyl-3-/2-(dimethylamino)-propyl/-3-phenyl-1H-indol-1-carboxaldehyd

Darstellung analog Beispiel 1, nur daß man (-)-2,3-Dihydro-2-methyl-3-/2-(dimethylamino)-propyl/-3-phenyl-1H-indol (ölige Base, beschrieben in der Deutschen Offenlegungsschrift 1 931 477, Beispiel 2c) anstatt des Racemats einsetzt.
Das Methansulfonat hat den Schmelzpunkt von 178-180 °C.

$[\alpha]_D^{20} = -97,4\,^{\circ}C$ (c = 1; 1 dm; Äthanol rein)

Pharmazeutische Beispiele

Die beanspruchte Verbindung und ihre Enantiomeren können zur pharmazeutischen Anwendung in die üblichen pharmazeutischen Präparate wie Kapseln, Tabletten und Briefchen zur Einzeldosis-Applikation eingearbeitet werden. Die Einzeldosis beträgt 5 bis 50 mg, bevorzugt 10 bis 25 mg, für Kinder etwa die Hälfte, die Tagesdosis 10 bis 200 mg, bevorzugt 20 bis 60 mg.

Beispiel I

Tabletten mit 20 mg Wirkstoff (Racemat der Formel I)
1 Tablette enthält:

| | |
|---|---|
| Wirkstoff | 20 mg |
| Milchzucker | 90 mg |
| Kartoffelstärke | 80 mg |
| Polyvinylpyrrolidon | 10 mg |
| Cellulose mikrokristallin | 23 mg |
| Magnesiumstearat | 2 mg |
| | 225 mg |

Herstellungsverfahren:
Die Mischung der Wirksubstanz mit Milchzucker und Kartoffelstärke wird mit einer 20 %igen Lösung des Polyvinylpyrrolidons gleichmäßig befeuchtet, durch ein Sieb der Maschenweite 1,5 mm granuliert und bei 35 °C getrocknet. Das trockene Granulat wird

nochmals durch dasselbe Sieb gerieben, mit den restlichen Hilfsstoffen gemischt und zu Tabletten verpreßt.
Tablettengewicht: 225 mg; Stempel: 9 mm, flach.

Beispiel II

Suppositorien mit 20 mg Wirkstoff
1 Zäpfchen enthält:

| | |
|---|---|
| Wirkstoff | 20 mg |
| Witepsol W 45® | 1600 mg |
| | 1620 mg |

Herstellungsverfahren:
Der fein gepulverte Wirkstoff wird in der geschmolzenen und auf 40°C abgekühlten Zäpfchenmasse suspendiert und in vorgekühlte Formen ausgegossen.
Zäpfchengewicht: 1,62 g

Beispiel III

Kapseln mit 20 mg Wirkstoff
1 Kapsel enthält:

| | |
|---|---|
| Wirkstoff | 20 mg |
| Milchzucker (trocken) | 200 mg |
| Magnesiumstearat | 2 mg |
| | 222 mg |

Herstellungsverfahren:
Der Wirkstoff wird mit dem trockenen Milchzucker und dem Magnesiumstearat gut gemischt und in Gelatinekapseln abgefüllt.

Beispiel IV

Einzeldosisbriefchen mit 20 mg Wirkstoff

Die Vorschrift für den Kapselinhalt aus Beispiel III wird übernommen. Das Wirkstoff-Hilfsstoff-Gemisch wird in Einzelbriefchen abgefüllt.

Patentansprüche:

1) Racemisches 2,3-Dihydro-2-methyl-3-[2-(dimethylamino)-propyl]-3-phenyl-1H-indol-1-carboxaldehyd der Formel I

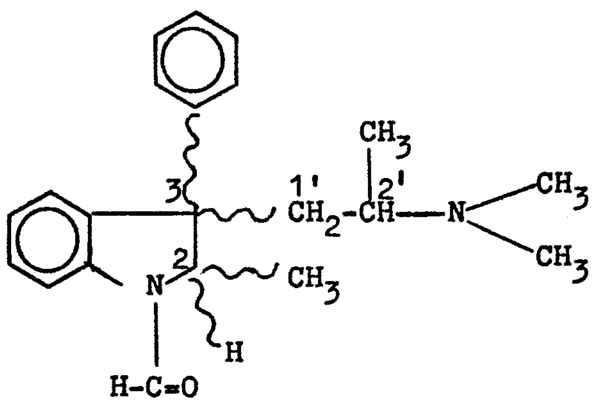

(I)

vom Schmelzpunkt 71-72°C, charakterisiert durch folgende $^{1}H$-NMR-Daten: ($d_6$-DMSO, 90 MHz, TMS als innerer Standard):

a) 400 K: $\delta$ = 8,63 (1H-s, CHO); 7,0 - 7,7 (9H-m, aromat. H); 4,65 (1H-q, 2-H, J = 6Hz); 1,76 - 2,5 (3H-m, Protonen in 1' und 2'); 2,03 (6H-s, $N(CH_3)_2$); 1,38 (3H-d, 2-$CH_3$, J = 6Hz); 0,66 (3H-d, $CH_3$ der Seitenkette, J = 6Hz)

b) 300 K: $\delta$ = 8,48 und 8,82 (jeweils s, zusammen 1H); 7,6 - 8,05 (1H-m, aromat. H); 7,0 - 7,5 (8H-m, aromat. H); 4,52 und 4,86 (jeweils q, zusammen 1H, J = 6Hz); 1,7 - 2,75 (9H-m); 1,37 und 1,22 (jeweils d, zusammen 3H, J = 6Hz); 0,5 - 0,8 (3H-m, evtl. Doppeldublett)

und dessen physiologisch verträgliche Salze mit anorganischen oder organischen Säuren.

2) Das (-)-Enantiomere der Verbindung der Formel I gemäß Anspruch 1.

3) Das (+)-Enantiomere der Verbindung der Formel I gemäß Anspruch 1.

4) Verfahren zur Herstellung des Racemats der Formel I, gemäß Anspruch 1 und seiner Enantiomeren gemäß den Ansprüchen 2 und 3 dadurch gekennzeichnet, daß
das entsprechende 2,3-Dihydro-2-methyl-3-[2-(dimethylamino)-propyl]-3-phenyl-1H-indol der Formel II

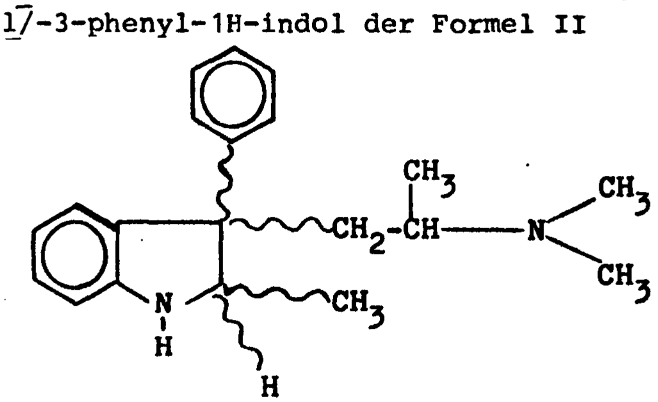

(II)

formyliert wird und gegebenenfalls die so erhaltene Verbindung in ihr physiologisch verträgliches Salz mittels anorganischer oder organischer Säuren übergeführt wird.

5) Verfahren zur Herstellung des Racemats der Formel I gemäß Anspruch 1 dadurch gekennzeichnet, daß das 3H-Indol der Formel III

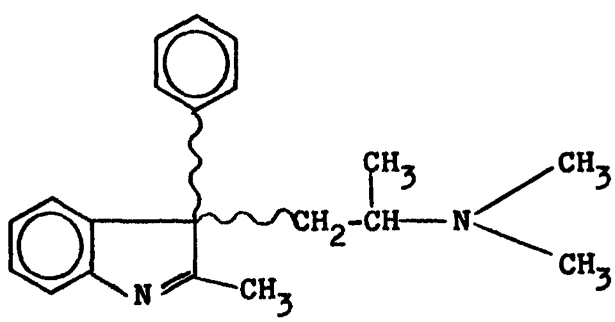

(III)

in Gegenwart eines Edelmetallkatalysators mit Wasserstoff und Ameisensäure reduktiv formyliert wird.

6) Arzneimittel enthaltend das Racemat der Formel I gemäß Anspruch 1 und/oder dessen Enantiomere neben den üblichen pharmazeutischen Trägern und Hilfsstoffen.

Europäisches Patentamt

# EUROPÄISCHER RECHERCHENBERICHT

Nummer der Anmeldung
EP 79 10 1438

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| D | DE - A - 1 931 477 (K. THOMAE GmbH)<br>* Patentansprüche *<br>-- | 1-6 | C 07 D 209/16<br>A 61 K 31/40 |
| D | DE - A - 1 470 370 (K. THOMAE GmbH)<br>* Patentansprüche *<br>---- | 1-6 | |

RECHERCHIERTE SACHGEBIETE (Int. Cl.²)

C 07 D 209/14
209/16

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 07-09-1979 | MAISONNEUVE |

EPA form 1503.1 06.78